# EUROPEAN PATENT APPLICATION

(11) **EP 1 130 122 A2**
(43) Date of publication of application: **05.09.2001**
(21) Application number: 01301291.9
(22) Date of filing: 13.02.2001
(51) Int. Cl.: C12Q 1/68, G01N 33/53, C07K 16/28, C07K 14/72

(54) **Methods for the diagnosis of polymorphisms in the human EP1-R gene**

(30) Priority: 17.02.2000 GB 0003553; 06.04.2000 GB 0008376
(71) Applicant: AstraZeneca AB, 151 85 Södertälje (SE)
(72) Inventor: Smith, John Craig, Macclesfield, Cheshire, SK10 4TG (GB); Anand, Rakesh, Macclesfield, Cheshire, SK10 4TG (GB); Morten, John Edward Norris, Macclesfield, Cheshire, SK10 4TG (GB)
(74) Representative: Gainey, Laurence David Scott

(57) **Abstract**

This invention relates to polymorphisms in the human prostaglandin E2 receptor 1 (EP1-R) gene and corresponding novel allelic polypeptides encoded thereby. Fourteen specific polymorphisms are identified. The invention also relates to methods and materials for analysing allelic variation in the EP1-R gene and to the use of said polymorphism in the diagnosis and treatment of EP1-R ligand mediated diseases, such as cancer or arthritis.

## Description

This invention relates to polymorphisms in the human prostaglandin E2 receptor 1 (EP1-R) gene and to corresponding novel allelic peptides encoded thereby. The invention also relates to methods and materials for analysing allelic variation in the EP1-R gene, and to the use of EP1-R polymorphism in the diagnosis and treatment of diseases in which modulation of EP1-R activity could be of therapeutic benefit, particularly disease states associated with pain such as rheumatoid arthritis, osteoarthritis and osteoporosis.

Prostaglandins (prostaglandin D2, E2, F2 alpha and I2) and thromboxane A2 (TXA2) are members of a family of hormones termed prostanoids, formed during the metabolism of arachidonic acid by cyclooxygenases. Prostanoids can be produced by many tissues and cells in response to a variety of stimuli, show a wide range of effects and are involved in regulation of many biological functions. They display a broad spectrum of biological properties which include contraction and relaxation of smooth muscle (including blood vessels, bronchi, uterus, gastrointestinal tract), inhibition of gastric acid secretion, and effects on platelet aggregation and endocrine and metabolic processes.

The biological actions of prostanoids are mediated through specific G-protein coupled cell surface receptors. The EP1 receptor (EP1-R) is one of four receptor subtypes termed EP1, EP2, EP3 and EP4, which mediate the biological activity of prostaglandin E2 (PGE2) (Negishi M *et al*., J Lipid Mediators Cell Signalling 12, 379-391, 1995). Each of the receptor subtypes possesses a distinct physiological role; each binds PGE2 with high affinity but displays differences in binding affinity for various PGE2 agonists and antagonists, and each mediates its effects via different signal transduction pathways (Coleman R *et al.*, Pharmacological Reviews 46, 205-229, 1994).

The EP1 receptor has been located on a wide range of tissues including stomach, small intestine, kidney, eye, uterus, trachea and muscle, and has been specifically related to induction of pain (Syriatowicz J *et al.*, Neuroscience 94, 587-594, 1999), fever (Oka K *et al.*, Am J Physiol 275/6 44-46, 1998), diuresis and osmoregulation (Breyer *et al*., Current Opinion in Nephrology and Hypertension 9/1, 23-29, 2000), initiation of labour (Spaziani *et al*., Biology of Reproduction 62/1, 23-26, 2000), and colon carcinogenesis (Watanabe *et al.,* Cancer Research 59/20 5093-5096, 1999).

Drugs which change the level of an EP1-R mediated response or change the biological activity of the EP1-R are useful in the treatment of all conditions in which the EP1-R plays a pathophysiological role. Such drugs are particularly useful in the treatment of conditions associated with pain, for example the pain associated with joint conditions (such as rheumatoid arthritis and osteoarthritis), post-operative pain, post-partum pain, the pain associated with dental conditions (such as dental caries and gingivitis), the pain associated with burns (including sunburn), the treatment of bone disorders (such as osteoporosis, hypercalcaemia of malignancy and Paget's disease), and the pain associated with sports injuries and sprains.

Examples of EP1-R drugs are given in WO 97/00864 Zeneca Ltd. and WO 96/03380 Zeneca Ltd.

A cDNA sequence encoding EP1-R has been cloned by Funk *et al*., J. Biol. Chem. 268, 26767-26772, 1993. The cDNA sequence has been submitted to the EMBL database under accession number L22647. The chromosomal location of the EP1-R gene has been mapped to 19p13.1 (Duncan *et al*., Genomics 25, 740-742, 1995).

The genomic DNA sequence of the human EP1-R gene has been published in the EMBLNEW database under accession number AC008569. The database entry incorrectly assigns the chromosomal location of the human EP1-R to chromosome 16.

Fragments of EP1-R genomic DNA sequence corresponding to positions 1-374, 732-2274 and 2276-3908 of SEQ ID NO.1 have been published under EMBL accession number AC008569. This database entry confirms the chromosomal location of the human EP1-R gene on chromosome 19.

We have determined the full length genomic DNA sequence of the human EP1-R gene, disclosed in SEQ ID NO.1 and shown in diagrammatic form in Figure 1. By comparing the genomic sequence of SEQ ID NO.1 with the cDNA sequence of L22647, we have identified some of the structural features of the EP1-R gene. There is an untranslated first exon at the 5' end of the gene. Exon 1 positions 1074-1130 in SEQ ID NO.1 correspond to positions 1-57 as defined in EMBL accession number L22647. There is a first intron at positions 1131-2054 of SEQ ID NO.1 followed by exon 2 at positions 2055-3013 of SEQ ID NO.1. The sequence of exon 2 corresponds to sequence positions 58-1016 as defined in EMBL accession number L22647. Exon 2 contains the initiating ATG codon at positions 2072-2074 of SEQ ID NO.1, corresponding to positions 75-78 as defined in EMBL accession number L22647. A second intron spans positions 3014-3565 of SEQ ID NO.1. Exon 3 is found at positions 3566-3908 of SEQ ID NO.1, corresponding to positions 1017-1359 as defined in EMBL accession number L22647. The intron/exon boundaries disclosed in SEQ ID NO.1 are consistent with the AG/GT consensus sequence. A comparison of the sequences disclosed in SEQ ID No.1 and EMBL accession number L22647 is shown in the Examples.

All positions in the human EP1-R gene herein refer to the positions in SEQ ID NO.1 unless stated otherwise or apparent from the context.

DNA polymorphisms are variations in DNA sequence between one individual and another. DNA polymorphisms may lead to variations in amino acid sequence and consequently to altered protein structure and functional activity. Polymorphisms may also affect mRNA synthesis, maturation, transportation and stability. Polymorphisms which do not result in amino acid changes (silent polymorphisms) or which do not alter any known consensus sequences may nevertheless have a biological effect, for example by altering mRNA folding, stability, splicing, transcription rate, translation rate, or fidelity. Recently, it has been reported that even polymorphisms that do not result in an amino acid change can cause different structural folds of mRNA with potentially different biological functions (Shen *et al*., (1999) Proc Nat1 Acad Sci USA **96**:7871-7876).

WO 00/29614 (Eurona Medical Labs) - published after the priority dates of the present application - also discloses the identification of various polymorphisms in the EP1-R gene. Based on the published EP1-R sequence, their analysis predicts the existence of eight EP1-R polymorphisms. The present analysis has identified probable errors in the original published sequence for EP1-R as all individuals analysed differed from the published sequence at nucleotides 285, 763 and 764 of L22647. This suggests that the predicted polymorphisms at positions 211, 689 and 690 in WO 00/29614 reflect the existence of errors in the original published sequence for EP1-R gene rather than true polymorphisms.

Knowledge of polymorphisms may be used to help identify patients most suited to therapy with particular pharmaceutical agents (this is often termed "pharmacogenetics"). Pharmacogenetics can also be used in pharmaceutical research to assist the drug selection process. Polymorphisms may be used in mapping the human genome and to elucidate the genetic component of diseases. The reader is directed to the following references for background details on pharmacogenetics and other uses of polymorphism detection: Linder *et al.* (1997), Clinical Chemistry, 43, 254; Marshall (1997), Nature Biotechnology, 15, 1249; International Patent Application WO 97/40462, Spectra Biomedical; and Schafer *et al.* (1998), Nature Biotechnology, 16, 33.

Clinical trials have shown that patient response to treatment with pharmaceuticals is often heterogeneous. Thus there is a need for improved approaches to pharmaceutical agent design and therapy.

The present invention is based on the discovery of fourteen polymorphisms in the human EP1-R gene.

According to a first aspect of the present invention there is provided a method for the diagnosis of a polymorphism in an EP1-R gene in a human, which method comprises determining the sequence of the nucleic acid of the human at one or more of positions 344, 621-627, 793-799, 908, 1136, 1160, 1189, 1458, 1656, 2448, 2531, 3348, 3432 and 3622 in the EP1-R gene as defined by the positions in SEQ ID NO.1; and determining the status of the human by reference to polymorphism in the EP1-R gene.

The term human includes both a human having or suspected of having an EP1-R mediated disease and an asymptomatic human who may be tested for predisposition or susceptibility to such disease. At each position the human may be homozygous for an allele or the human may be a heterozygote.

The term 'EP1-R mediated disease' means any disease in which changing the level of an EP1-R mediated response or changing the biological activity of the EP1-R would be of therapeutic benefit.

The term 'EP1-R drug' means any drug which changes the level of an EP1-R mediated response or changes the biological activity of the EP1-R. For example the drug may be an agonist or an antagonist of a natural ligand for the EP1-R. A drug which inhibits the activity of the EP1-R is preferred.

Variations in polypeptide sequence will be referred to as follows: original amino acid (using one or three letter nomenclature), position, new amino acid. For a hypothetical example "D25K" or "Asp25Lys" means that at position 25 an aspartic acid has been changed to lysine.

The term polymorphism includes single nucleotide substitution, nucleotide insertion and nucleotide deletion, which in the case of insertion and deletion includes insertion or deletion of one or more nucleotides at a position of a gene and variable numbers of a repeated DNA sequence.

In one embodiment of the invention preferably the method for diagnosis described herein is one in which the single nucleotide polymorphism at position 344 in the EP1-R gene as defined by the positions in SEQ ID NO.1 is presence of G and/or A.

In another embodiment of the invention preferably the method for diagnosis described herein is one in which the polymorphism at position 621-627 in the EP1-R gene as defined by the positions in SEQ ID NO.1 is deletion of one or more of the seven Gs. Preferably allelic variation consists of a single nucleotide deletion of one of the seven Gs.

In another embodiment of the invention preferably the method for diagnosis described herein is one in which the polymorphism at position 793-799 in the EP1-R gene as defined by the positions in SEQ ID NO.1 is deletion of one or more of the seven Gs. Preferably allelic variation consists of a single nucleotide deletion of one of the seven Gs.

In another embodiment of the invention preferably the method for diagnosis described herein is one in which the polymorphism at position 908 in the EP1-R gene as defined by the positions in SEQ ID NO.1 is presence of C and/or T.

In another embodiment of the invention preferably the method for diagnosis described herein is one in which the polymorphism at position 1136 in the EP1-R gene as defined by the positions in SEQ ID NO.1 is presence of G and/or C.

In another embodiment of the invention preferably the method for diagnosis described herein is one in which the polymorphism at position 1160 in the EP1-R gene as defined by the positions in SEQ ID NO.1 is presence of T and/or C.

In another embodiment of the invention preferably the method for diagnosis described herein is one in which the polymorphism at position 1189 in the EP1-R gene as defined by the positions in SEQ ID NO.1 is presence of G and/or A.

In another embodiment of the invention preferably the method for diagnosis described herein is one in which the polymorphism at position 1458 in the EP1-R gene as defined by the positions in SEQ ID NO.1 is presence of A and/or G.

In another embodiment of the invention preferably the method for diagnosis described herein is one in which the polymorphism at position 1656 in the EP1-R gene as defined by the positions in SEQ ID NO.1 is presence of T and/or G.

In another embodiment of the invention preferably the method for diagnosis described herein is one in which the polymorphism at position 2448 in the EP1-R gene as defined by the positions in SEQ ID NO.1 is presence of T and/or C.

In another embodiment of the invention preferably the method for diagnosis described herein is one in which the polymorphism at position 2531 in the EP1-R gene as defined by the positions in SEQ ID NO.1 is presence of G and/or A.

In another embodiment of the invention preferably the method for diagnosis described herein is one in which the polymorphism at position 3348 in the EP1-R gene as defined by the positions in SEQ ID NO.1 is presence of C and/or T.

In another embodiment of the invention preferably the method for diagnosis described herein is one in which the polymorphism at position 3432 in the EP1-R gene as defined by the positions in SEQ ID NO.1 is presence of C and/or G.

In another embodiment of the invention preferably the method for diagnosis described herein is one in which the polymorphism at position 3622 in the EP1-R gene as defined by the positions in SEQ ID NO.1 is presence of G and/or A.

In another embodiment of the invention preferably the method for diagnosis described herein is one selected from the group in which, as defined by the positions in SEQ ID NO.1: the polymorphism at position 344 in the EP1-R gene is presence of G and/or A, the polymorphism at position 621-627 in the EP1-R gene is deletion of one or more of the seven Gs, the polymorphism at position 793-799 in the EP1-R gene is deletion of one or more of the seven Gs, the polymorphism at position 908 in the EP1-R gene is presence of C and/or T, the polymorphism at position 1136 in the EP1-R gene is presence of G and/or C, the polymorphism at position 1160 in the EP1-R gene is presence of T and/or C, the polymorphism at position 1189 in the EP1-R gene is presence of G and/or A, the polymorphism at position 1458 in the EP1-R gene is presence of A and/or G, the polymorphism at position 1656 in the EP1-R gene is presence of T and/or G, the polymorphism at position 2448 in the EP1-R gene is presence of T and/or C, the polymorphism at position 2531 in the EP1-R gene is presence of G and/or A, the polymorphism at position 3348 in the EP1-R gene is presence of C and/or T, the polymorphism at position 3432 in the EP1-R gene is presence of C and/or G and, the polymorphism at position 3622 in the EP1-R gene is presence of G and/or A.

It will be appreciated by the person skilled in the art that the numbering of the nucleotide positions in the EP1-R gene will vary according to the number of deletions at positions 621-627 and 793-799 as defined by the sequence in SEQ ID NO.1. For example, in a first allele comprising 7xG nucleotides at positions 621-627 of SEQ ID NO.1 the nucleotide sequence immediately following the 7xG will start at position 628. In a second allele where one of the G's at positions 621-627 of SEQ ID NO.1 is deleted, the nucleotide sequence immediately following the remaining 6xG's will now start at position number 627 and so forth.

The method for diagnosis is preferably one in which the sequence is determined by a method selected from amplification refractory mutation system (ARMS™-allele specific amplification), allele specific hybridisation (ASH), oligonucleotide ligation assay (OLA) and restriction fragment length polymorphism (RFLP). The amino acid sequence method for diagnosis is preferably one which is determined by immunological methods such as enzyme linked immunosorbent assay (ELISA).

In another aspect of the invention there is provided a method of analysing a nucleic acid, comprising: obtaining a nucleic acid from an individual; and determining the base occupying any one of the following polymorphic sites: 344, 621-627, 793-799, 908, 1136, 1160, 1189, 1458, 1656, 2448, 2531, 3348, 3432 and 3622 in the EP1-R gene as defined by the positions in SEQ ID NO.1.

In another aspect of the invention we provide a method for the diagnosis of EP1-R-mediated disease, which method comprises:
i) obtaining sample nucleic acid from an individual,
ii) detecting the presence or absence of a variant nucleotide at one or more of positions 344, 621-627, 793-799, 908, 1136, 1160, 1189, 1458, 1656, 2448, 2531, 3348, 3432 and 3622 in the EP1-R gene as defined by the positions in SEQ ID NO.1; and
iii) determining the status of the individual by reference to polymorphism in the EP1-R gene.

Allelic variation at each position in the EP1-R gene, including preferred variation is described herein.

The status of the individual may be determined by reference to allelic variation at any one, two, three, four, five, six, seven, eight, nine, ten, eleven, twelve, thirteen or all fourteen positions optionally in combination with any other polymorphism in the gene that is (or becomes) known.

The test sample of nucleic acid is conveniently present in a sample of blood, sputum, skin, bronchoalveolar lavage fluid, or other body fluid or tissue obtained from an individual. It will be appreciated that the test sample may equally comprise a nucleic acid sequence corresponding to the sequence in the test sample, that is to say that all or a part of the region in the sample nucleic acid may firstly be amplified using any convenient technique e.g. PCR, before analysis of allelic variation.

It will be apparent to the person skilled in the art that there are a large number of analytical procedures which may be used to detect the presence or absence of variant nucleotides at one or more polymorphic positions of the invention. In general, the detection of allelic variation requires a mutation discrimination technique, optionally an amplification reaction and optionally a signal generation system. Table 1 lists a number of mutation detection techniques, some based on PCR. These may be used in combination with a number of signal generation systems, a selection of which is listed in Table 2. Further amplification techniques are listed in Table 3. Many current methods for the detection of allelic variation are reviewed by Nollau *et al.*, Clin. Chem. 43, 1114-1120, 1997; and in standard textbooks, for example "Laboratory Protocols for Mutation Detection", Ed. by U. Landegren, Oxford University Press, 1996 and "PCR", 2^{nd} Edition by Newton & Graham, BIOS Scientific Publishers Limited, 1997.

| **Abbreviations:** | |
|---|---|
| ALEX™ | Amplification refractory mutation system linear extension |
| APEX | Arrayed primer extension |
| ARMS™ | Amplification refractory mutation system |
| ASA | Allele specific amplification |
| b-DNA | Branched DNA |
| CMC | Chemical mismatch cleavage |
| COPS | Competitive oligonucleotide priming system |
| DGGE | Denaturing gradient gel electrophoresis |
| FRET | Fluorescence resonance energy transfer |
| LCR | Ligase chain reaction |
| MASDA | Multiple allele specific diagnostic assay |
| NASBA | Nucleic acid sequence based amplification |
| OLA | Oligonucleotide ligation assay |
| PCR | Polymerase chain reaction |
| PTT | Protein truncation test |
| RFLP | Restriction fragment length polymorphism |
| SDA | Strand displacement amplification |
| SERRS | Surface enhanced raman resonance spectroscopy |
| SNP | Single nucleotide polymorphism |
| SSCP | Single-strand conformation polymorphism analysis |
| SSR | Self sustained replication |
| TGGE | Temperature gradient gel electrophoresis |

### Table 1 - Mutation Detection Techniques

**General**: DNA sequencing, Sequencing by hybridisation
**Scanning**: PTT*, SSCP, DGGE, TGGE, Cleavase, Heteroduplex analysis, CMC, Enzymatic mismatch cleavage
* Note: not useful for detection of promoter polymorphisms.
**Hybridisation Based:**
Solid phase hybridisation: Dot blots, MASDA, Reverse dot blots, Oligonucleotide arrays
(DNA Chips).
Solution phase hybridisation: Taqman™ - US-5210015 & US-5487972 (Hoffmann-La Roche), Molecular Beacons - Tyagi *et al* (1996), Nature Biotechnology, **14**, 303; WO 95/13399 (Public Health Inst., New York).
**Extension Based:** ARMS™-allele specific amplification (as described in European patent No. EP-B-332435 and US patent No. 5,595,890), ALEX™ - European Patent No. EP 332435 B1 (Zeneca Limited), COPS - Gibbs *et al* (1989), Nucleic Acids Research, **17**, 2347. **Incorporation Based:** Mini-sequencing, APEX
**Restriction Enzyme Based:** RFLP, Restriction site generating PCR
**Ligation Based:** OLA
**Other:** Invader assay

### Table 2 - Signal Generation or Detection Systems

**Fluorescence:** FRET, Fluorescence quenching, Fluorescence polarisation - United Kingdom Patent No. 2228998 (Zeneca Limited)
**Other:** Chemiluminescence, Electrochemiluminescence, Raman, Radioactivity, Colorimetric, Hybridisation protection assay, Mass spectrometry and SERRS - WO 97/05280 (University of Strathclyde).

### Table 3 - Further Amplification Methods

SSR, NASBA, LCR, SDA, b-DNA

Preferred mutation detection techniques include ARMS™-ASA, ALEX™, COPS, Taqman, Molecular Beacons, RFLP, restriction site based PCR and FRET techniques, polyacrylamide gel electrophoresis and capillary electrophoresis.

Particularly preferred methods include ARMS™-ASA and RFLP based methods. ARMS™-ASA is an especially preferred method.

ARMS™-allele specific amplification (described in European patent No. EP-B-332435, US patent No. 5,595,890 and Newton et al. (Nucleic Acids Research, Vol. 17, p.2503; 1989)), relies on the complementarity of the 3' terminal nucleotide of the primer and its template. The 3' terminal nucleotide of the primer being either complementary or non-complementary to the specific mutation, allele or polymorphism to be detected. There is a selective advantage for primer extension from the primer whose 3' terminal nucleotide complements the base mutation, allele or polymorphism. Those primers which have a 3' terminal mismatch with the template sequence severely inhibit or prevent enzymatic primer extension. Polymerase chain reaction or unidirectional primer extension reactions therefore result in product amplification when the 3' terminal nucleotide of the primer complements that of the template, but not, or at least not efficiently, when the 3' terminal nucleotide does not complement that of the template.

In a further aspect, the diagnostic methods of the invention are used to assess the efficacy of therapeutic compounds in the treatment of EP1-R-mediated diseases particularly disease states associated with pain such as rheumatoid arthritis, osteoarthritis and osteoporosis.

Assays, for example reporter-based assays, may be devised to detect whether one or more of the above polymorphisms affect transcription levels and/or message stability.

The polymorphisms identified in the present invention that occur in intron regions or in the promoter region are not expected to alter the amino acid sequence of the EP1-receptor, but may affect the transcription and/or message stability of the sequences and thus affect the level of the receptors in cells.

Two of the polymorphisms of the present invention result in variations in amino acid sequence in the translated protein. Polymorphism at position 2448 as defined in SEQ ID NO.1 results in an amino acid change from leucine to proline at corresponding position 126 of the translated protein (Leul26Pro). Polymorphism at position 2531 as defined in SEQ ID NO.1 results in an amino acid change from alanine to threonine at corresponding position 154 of the translated protein (A1a154Thr). Changes in protein sequence may be detected using standard techniques known to the person skilled in the art such as immunoassay techniques that employing specific antibodies capable of discriminating peptide regions that differ by one or more amino acids. Detection of the amino acid changing polymorphisms also forms part of this invention.

Thus, according to a further aspect of the invention there is provided a method for detecting the presence of a polymorphism in the EP1-R protein in a human, which method comprises determining the amino acid present at one or both of amino acid positions 126 and 154 of the EP1-R protein; and determining the status of the individual by reference to the amino acid detected.

In another aspect of the invention there is provided a method for the diagnosis of EP1-R-mediated disease, which method comprises:
i) obtaining a protein containing sample from an individual;
ii) detecting the presence or absence of a variant EP1-R polypeptide on the basis of the presence of a polymorphic amino acid at either or both amino acid positions: 126 and 154; and,
iii) determining the status of the human by reference to the presence or absence of a polymorphism in EP1-R protein.

In a preferred embodiment the polymorphic amino acid at position 126 is presence of proline and at position 154 is presence of threonine.

Individuals who carry particular allelic variants of the EP1-R gene may exhibit differences in their ability to regulate protein biosynthesis under different physiological conditions and may display altered abilities to react to different diseases. In addition, differences in protein regulation and/or the protein's properties arising as a result of allelic variation may have a direct effect on the response of an individual to drug therapy. The diagnostic methods of the invention may be useful both to predict the clinical response to such agents and to determine therapeutic dose.

In a further aspect, the diagnostic methods of the invention, are used to assess the predisposition of an individual to diseases mediated by EP1-R. This may be particularly relevant in the development of pain and in diseases which are mediated by EP1-R. The present invention may be used to recognise individuals who are particularly at risk from developing these conditions.

Low frequency polymorphisms may be particularly useful for haplotyping as described below. A haplotype is a set of alleles found at linked polymorphic sites (such as within a gene) on a single (paternal or maternal) chromosome. If recombination within the gene is random, there may be as many as 2ⁿ haplotypes, where 2 is the number of alleles at each polymorphic position and n is the number of polymorphic positions. One approach to identifying mutations or polymorphisms which are correlated with clinical response is to carry out an association study using all the haplotypes that can be identified in the population of interest. The frequency of each haplotype is limited by the frequency of its rarest allele, so that polymorphisms with low frequency alleles are particularly useful as markers of low frequency haplotypes. As particular mutations or polymorphisms associated with certain clinical features, such as adverse or abnormal events, are likely to be of low frequency within the population, low frequency polymorphisms may be particularly useful in identifying these mutations (for examples see: De Stefano V et al., *Ann Hum Genet* (1998) 62:481-90; and Keightley AM et al., *Blood* (1999) **93**:4277-83.

In a further aspect, the diagnostic methods of the invention are used in the development of new drug therapies which selectively target one or more allelic variants of the EP1-R gene. Identification of a link between a particular allelic variant and predisposition to disease development or response to drug therapy may have a significant impact on the design of new drugs. Drugs may be designed to regulate the biological activity of variants implicated in the disease process whilst minimising effects on other variants.

In a further diagnostic aspect of the invention the presence or absence of variant nucleotides is detected by reference to the loss or gain of, optionally engineered, sites recognised by restriction enzymes (see Example 3).

According to another aspect of the present invention there is provided a nucleic acid comprising any one of the following polymorphisms:
the nucleic acid of SEQ ID NO.1 with A at position 344 as defined by the positions in SEQ ID NO.1;
the nucleic acid of SEQ ID NO.1 with six Gs at positions 621-626 as defined by the positions in SEQ ID NO.1;
the nucleic acid of SEQ ID NO.1 with six Gs at positions 793-798 as defined by the positions in SEQ ID NO.1;
the nucleic acid of SEQ ID NO.1 with T at position 908 as defined by the positions in SEQ ID NO.1;
the nucleic acid of SEQ ID NO.1 with C at position 1136 as defined by the positions in SEQ ID NO.1;
the nucleic acid of SEQ ID NO.1 with C at position 1160 as defined by the positions in SEQ ID NO.1;
the nucleic acid of SEQ ID NO.1 with A at position 1189 as defined by the positions in SEQ ID NO.1;
the nucleic acid of SEQ ID NO.1 with G at position 1458 as defined by the positions in SEQ ID NO.1;
the nucleic acid of SEQ ID NO.1 with G at position 1656 as defined by the positions in SEQ ID NO.1;
the nucleic acid of SEQ ID NO.1 with C at position 2448 as defined by the positions in SEQ ID NO.1;
the nucleic acid of SEQ ID NO.1 with A at position 2531 as defined by the positions in SEQ ID NO.1;
the nucleic acid of SEQ ID NO.1 with T at position 3348 as defined by the positions in SEQ ID NO.1;
the nucleic acid of SEQ ID NO.1 with G at position 3432 as defined by the positions in SEQ ID NO.1;
the nucleic acid of SEQ ID NO.1 with A at position 3622 as defined by the positions in SEQ ID NO.1;
or a complementary strand thereof or an antisense sequence thereto or a fragment thereof of at least 17 bases comprising at least one polymorphism.

Fragments are at least 17 bases, more preferably at least 20 bases, more preferably at least 30 bases.

A nucleic acid of the invention is preferably in isolated form, for example through being at least partially purified from any substance with which it occurs naturally (if any).

Novel sequence disclosed herein, may be used in another embodiment of the invention to regulate expression of the gene in cells by the use of antisense constructs. To enable methods of down-regulating expression of the gene of the present invention in mammalian cells, an example antisense expression construct can be readily constructed for instance using the pREP10 vector (Invitrogen Corporation). Transcripts are expected to inhibit translation of the gene in cells transfected with this type of construct. Antisense transcripts are effective for inhibiting translation of the native gene transcript, and capable of inducing the effects (e.g., regulation of tissue physiology) herein described. Oligonucleotides which are complementary to and hybridisable with any portion of novel gene mRNA disclosed herein are contemplated for therapeutic use. U.S. Patent No. 5,639,595, "Identification of Novel Drugs and Reagents", issued Jun. 17, 1997, wherein methods of identifying oligonucleotide sequences that display in vivo activity are thoroughly described, is herein incorporated by reference. Expression vectors containing random oligonucleotide sequences derived from previously known polynucleotides are transformed into cells. The cells are then assayed for a phenotype resulting from the desired activity of the oligonucleotide. Once cells with the desired phenotype have been identified, the sequence of the oligonucleotide having the desired activity can be identified. Identification may be accomplished by recovering the vector or by polymerase chain reaction (PCR) amplification and sequencing the region containing the inserted nucleic acid material. Antisense molecules can be synthesised for antisense therapy. These antisense molecules may be DNA, stable derivatives of DNA such as phosphorothioates or methylphosphonates, RNA, stable derivatives of RNA such as 2'-O-alkylRNA, or other oligonucleotide mimetics. U.S. Patent No. 5,652,355, "Hybrid Oligonucleotide Phosphorothioates", issued July 29, 1997, and U.S. Patent No. 5,652,356, "Inverted Chimeric and Hybrid Oligonucleotides", issued July 29, 1997, which describe the synthesis and effect of physiologically-stable antisense molecules, are incorporated by reference. Antisense molecules may be introduced into cells by microinjection, liposome encapsulation or by expression from vectors harboring the antisense sequence.

The invention further provides nucleotide primers which can detect the polymorphisms of the invention.

According to another aspect of the present invention there is provided an allele specific primer capable of detecting an EP1-R gene polymorphism at one or more of positions 344, 621-627, 793-799, 908, 1136, 1160, 1189, 1458, 1656, 2448, 2531, 3348, 3432, and 3622 in the EP1-R gene as defined by the positions in SEQ ID NO.1.

An allele specific primer is used, generally together with a constant primer, in an amplification reaction such as a PCR reaction, which provides the discrimination between alleles through selective amplification of one allele at a particular sequence position e.g. as used for ARMS™-allele specific amplification assays. The allele specific primer is preferably 17-50 nucleotides, more preferably about 17-35 nucleotides, more preferably about 17-30 nucleotides.

An allele specific primer preferably corresponds exactly with the allele to be detected but derivatives thereof are also contemplated wherein about 6-8 of the nucleotides at the 3' terminus correspond with the allele to be detected and wherein up to 10, such as up to 8, 6, 4, 2, or 1 of the remaining nucleotides may be varied without significantly affecting the properties of the primer.

Primers may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1^{st} Edition. If required the primer(s) may be labelled to facilitate detection.

According to another aspect of the present invention there is provided an allele-specific oligonucleotide probe capable of detecting an EP1-R gene polymorphism at one or more of positions 344, 621-627, 793-799, 908, 1136, 1160, 1189, 1458, 1656, 2448, 2531, 3348, 3432 and 3622 in the EP1-R gene as defined by the positions in SEQ ID NO.1.

The allele-specific oligonucleotide probe is preferably 17- 50 nucleotides, more preferably about 17-35 nucleotides, more preferably about 17-30 nucleotides.

The design of such probes will be apparent to the molecular biologist of ordinary skill. Such probes are of any convenient length such as up to 50 bases, up to 40 bases, more conveniently up to 30 bases in length, such as for example 8-25 or 8-15 bases in length. In general such probes will comprise base sequences entirely complementary to the corresponding wild type or variant locus in the gene. However, if required one or more mismatches may be introduced, provided that the discriminatory power of the oligonucleotide probe is not unduly affected. The probes of the invention may carry one or more labels to facilitate detection.

Oligonucleotide probes and primers generally differ according to the location of the base capable of hybridising with the polymorphic base. Thus, with a probe, the nucleotide capable of complementing the polymorphism is located in a centralised position, whereas with a primer (to be used in an amplification reaction) the nucleotide complementing the polymorphism is preferably located at the 3' end of the primer.

According to another aspect of the present invention there is provided a diagnostic kit comprising an allele specific oligonucleotide probe of the invention and/or an allele-specific primer of the invention.

The diagnostic kits may comprise appropriate packaging and instructions for use in the methods of the invention. Such kits may further comprise appropriate buffer(s), nucleotides, and polymerase(s) such as thermostable polymerases, for example taq polymerase.

In another aspect of the invention, the polymorphisms of this invention may be used as genetic markers in linkage studies. This particularly applies to the polymorphisms at positions 621-627, 1136, 1189, 1458, 3348 and 3622 in the EP1-R gene as defined by the positions in SEQ ID NO.1 because of their relatively high frequency (See Example 1).

According to another aspect of the present invention there is provided a method of treating a human in need of treatment with an EP1-R drug in which the method comprises:
i) diagnosis of a polymorphism in the EP1-R gene in the human, which diagnosis comprises determining the sequence of the nucleic acid at one or more of positions 344, 621-627, 793-799, 908, 1136, 1160, 1189, 1458, 1656, 2448, 2531, 3348, 3432 and 3622 in the EP1-R gene as defined by the positions in SEQ ID NO.1, and determining the status of the human by reference to polymorphism in the EP1-R gene; and
ii) administering an effective amount of a EP1-R drug.

Preferably determination of the status of the human is clinically useful. Examples of clinical usefulness include deciding which drug or drugs to administer and/or establishing the effective amount of the drug or drugs.

Drugs which decrease the activity of EP1-R are of value in a number of disease conditions, including disease states associated with pain such as rheumatoid arthritis, osteoarthritis and osteoporosis.

According to another aspect of the present invention there is provided use of an EP1-R drug in the preparation of a medicament for treating an EP1 R-mediated disease in a human diagnosed as having a polymorphism at one or more of positions 344, 621-627, 793-799, 908, 1136, 1160, 1189, 1458, 1656, 2448, 2531, 3348, 3432 and 3622 in the EP1-R gene as defined by the positions in SEQ ID NO.1.

According to another aspect of the present invention there is provided a pharmaceutical pack comprising an EP1-R drug and instructions for administration of the drug to humans diagnostically tested for a polymorphism at one or more of positions 344, 621-627, 793-799, 908, 1136, 1160, 1189, 1458, 1656, 2448, 2531, 3348, 3432 and 3622 in the EP1-R gene as defined by the positions in SEQ ID NO.1.

According to another aspect of the present invention there is provided a computer readable medium comprising at least one novel polynucleotide sequence of the invention stored on the medium. The computer readable medium may be used, for example, in homology searching, mapping, haplotyping, genotyping or pharmacogenetic analysis or any other bioinformatic analysis. The reader is referred to Bioinformatics, A practical guide to the analysis of genes and proteins, Edited by A D Baxevanis & B F F Ouellette, John Wiley & Sons, 1998. Any computer readable medium may be used, for example, floppy disks, tapes, chips, compact disks, digital disks, video disks, punch cards and hard drives.

The polynucleotide sequences of the invention, or parts thereof, particularly those relating to and identifying the polymorphisms identified herein represent a valuable information source, for example, to characterise individuals in terms of haplotype and other sub-groupings, such as investigation of susceptibility to treatment with particular drugs. These approaches are most easily facilitated by storing the sequence information in a computer readable medium and then using the information in standard bioinformatics programs or to search sequence databases using state of the art searching tools such as "GCC" (Genetics Computer Group), BlastX, BlastP, BlastN, FASTA (refer to Altschul *et al.* (1990) J. Mol. Biol. **215**:403-410). Thus, the polynucleotide sequences of the invention are particularly useful as components in databases useful for sequence identity and other search analyses. As used herein, storage of the sequence information in a computer readable medium and use in sequence databases in relation to 'polynucleotide or polynucleotide sequence of the invention' covers any detectable chemical or physical characteristic of a polynucleotide of the invention that may be reduced to, converted into or stored in a tangible medium, such as a computer disk, preferably in a computer readable form. For example, chromatographic scan data or peak data, photographic scan or peak data, mass spectrographic data, sequence gel (or other) data.

The invention provides a computer readable medium having stored thereon one or more polynucleotide sequences of the invention. For example, a computer readable medium is provided comprising and having stored thereon a member selected from the group consisting of: a polynucleotide comprising the sequence of a polynucleotide of the invention, a polynucleotide consisting of a polynucleotide of the invention, a polynucleotide which comprises part of a polynucleotide of the invention, which part includes at least one of the polymorphisms of the invention, a set of polynucleotide sequences wherein the set includes at least one polynucleotide sequence of the invention, a data set comprising or consisting of a polynucleotide sequence of the invention or a part thereof comprising at least one of the polymorphisms identified herein.

Thus, according to another aspect of the invention there is provided a computer readable medium having stored thereon a nucleic acid sequence comprising at least 17, preferably at least 20 consecutive bases of the EP1-R gene sequence, which sequence includes at least one of the polymorphisms at positions: 344, 621-627, 793-799, 908, 1136, 1160, 1189, 1458, 1656, 2448, 2531, 3348, 3432 and 3622 in the EP1-R gene as defined by the positions in SEQ ID NO.1.

A computer based method is also provided for performing sequence identification, said method comprising the steps of providing a polynucleotide sequence comprising a polymorphism of the invention in a computer readable medium; and comparing said polymorphism containing polynucleotide sequence to at least one other polynucleotide or polypeptide sequence to identify identity (homology), i.e. screen for the presence of a polymorphism.

In another aspect of the invention there is provided a method for performing sequence identification, said method comprising the steps of providing a nucleic acid sequence comprising at least 20 consecutive bases of the EP1-R gene sequence, which sequence includes at least one of the polymorphisms at positions: 344, 621-627, 793-799, 908, 1136, 1160, 1189, 1458, 1656, 2448, 2531, 3348, 3432 and 3622 in the EP1-R gene as defined by the positions in SEQ ID NO.1, in a computer readable medium; and comparing said nucleic acid sequence to at least one other nucleic acid sequence to identify identity.

Two of the polymorphisms of the present invention result in variations in amino acid sequence in the translated protein. Polymorphism at position 2448 as defined in SEQ ID NO.1 results in an amino acid change from leucine to proline at corresponding position 126 of the translated protein (Leul26Pro). Polymorphism at position 2531 as defined in SEQ ID NO.1 results in an amino acid change from alanine to threonine at corresponding position 154 of the translated protein (A1a154Thr).

Thus according to another aspect of the present invention there is provided an allelic variant of the human EP1-R polypeptide having a proline at position 126 and/or a threonine at position 154 or a fragment thereof comprising at least 10 amino acids provided that the fragment comprises the allelic variant at position 126 and/or position 154. Preferably the allelic variant is at least 30% pure, more preferably at least 60% pure, more preferably at least 90% pure, more preferably at least 95% pure, and more preferably at least 99% pure.

Fragments of EP1-R polypeptide are at least 10 amino acids, more preferably at least 15 amino acids, more preferably at least 20 amino acids. The polypeptides of the invention do not encompass naturally occurring polypeptides as they occur in nature, for example, the polypeptide is at least partially purified from at least one component with which it occurs naturally. Preferably the polypeptide is at least 30% pure, more preferably at least 60% pure, more preferably at least 90% pure, more preferably at least 95% pure, and more preferably at least 99% pure.

According to another aspect of the present invention there is provided an antibody specific for an allelic variant of human EP1-R polypeptide having a proline at position 126 and/or a threonine at position 154 or a fragment thereof comprising at least 10 amino acids provided that the fragment comprises the allelic variants at position 126 and /or position 154.

Antibodies can be prepared using any suitable method. For example, purified polypeptide may be utilised to prepare specific antibodies. The term "antibodies" includes polyclonal antibodies, monoclonal antibodies, and the various types of antibody constructs such as for example F(ab')₂, Fab and single chain Fv. Antibodies are defined to be specifically binding if they bind the antigen with a Kₐ of greater than or equal to about 10⁷ M⁻¹. Affinity of binding can be determined using conventional techniques, for example those described by Scatchard et al., *Ann. N.Y. Acad. Sci.,* 51:660 (1949).

Polyclonal antibodies can be readily generated from a variety of sources, for example, horses, cows, goats, sheep, dogs, chickens, rabbits, mice or rats, using procedures that are well-known in the art. In general, antigen is administered to the host animal typically through parenteral injection. The immunogenicity of antigen may be enhanced through the use of an adjuvant, for example, Freund's complete or incomplete adjuvant. Following booster immunisations, small samples of serum are collected and tested for reactivity to antigen. Examples of various assays useful for such determination include those described in: *Antibodies: A Laboratory Manual,* Harlow and Lane (eds.), Cold Spring Harbor Laboratory Press, 1988; as well as procedures such as countercurrent immuno-electrophoresis (CIEP), radioimmunoassay, radioimmunoprecipitation, enzyme-linked immuno-sorbent assays (ELISA), dot blot assays, and sandwich assays, see U.S. Patent Nos. 4,376,110 and 4,486,530.

Monoclonal antibodies may be readily prepared using well-known procedures, see for example, the procedures described in U.S. Patent Nos. RE 32,011, 4,902,614, 4,543,439 and 4,411,993; Monoclonal Antibodies, Hybridomas: *A New Dimension in Biological Analyses,* Plenum Press, Kennett, McKearn, and Bechtol (eds.), (1980).

The monoclonal antibodies of the invention can be produced using alternative techniques, such as those described by Alting-Mees et al., "Monoclonal Antibody Expression Libraries: A Rapid Alternative to Hybridomas", *Strategies in Molecular Biology* 3: 1-9 (1990) which is incorporated herein by reference. Similarly, binding partners can be constructed using recombinant DNA techniques to incorporate the variable regions of a gene that encodes a specific binding antibody. Such a technique is described in Larrick et al., *Biotechnology,* 7: 394 (1989).

Once isolated and purified, the antibodies may be used to detect the presence of antigen in a sample using established assay protocols.

The invention will now be illustrated but not limited by reference to the following Examples and Figure. All temperatures are in degrees Celsius.

In the Examples below, unless otherwise stated, the following methodology and materials have been applied.

AMPLITAQ™ or AMPLITAQ GOLD™ available from Perkin-Elmer Cetus, are used as the source of thermostable DNA polymerase.

General molecular biology procedures can be followed from any of the methods described in "Molecular Cloning - A Laboratory Manual" Second Edition, Sambrook, Fritsch and Maniatis (Cold Spring Harbor Laboratory, 1989) or "Current Protocols in Molecular Biology Volumes1-3 ,Edited by FM Asubel, R Brent, RE Kingston pub John Wiley 1998.

Electropherograms were obtained in a standard manner: data was collected by ABI377 data collection software and the wave form generated by ABI Prism™ sequencing analysis (2.1.2).

### Figure 1

Genomic DNA sequence of the human EP1-R gene.

### Example 1

### Identification of Polymorphisms

### 1. Methods

### Genomic DNA Preparation

Genomic DNA was prepared from lymphoblastoid cell lines from Caucasian donors following protocol I (Molecular Cloning: A Laboratory Manual, p392, Sambrook, Fritsch and Maniatis, 2nd Edition, Cold Spring Harbor Press, 1989) with the following modifications. Samples were extracted with phenol, then phenol/chloroform and then chloroform rather than with three phenol extractions. The DNA was dissolved in deionised water.

### Template Preparation

Templates were prepared by PCR. The extension temperature was 72° and denaturation temperature 94°; each step was 1 minute. Generally 50 ng genomic DNA was used in each reaction and subjected to 40 cycles of PCR.

For dye-primer sequencing the forward primers were modified to include M13 forward sequence (ABI protocol P/N 402114, Applied Biosystems) at the 5' end of the oligonucleotides.

### Dye Primer Sequencing

Dye-primer sequencing using M13 forward primer was as described in the ABI protocol P/N 402114 for the ABI Prism™ dye primer cycle sequencing core kit with "AmpliTaq FS"™ DNA polymerase, modified in that the annealing temperature was 45° and DMSO was added to the cycle sequencing mix to a final concentration of 5%.

The extension reactions for each base were pooled, ethanol/sodium acetate precipitated, washed and resuspended in formamide loading buffer.

4.25% Acrylamide gels were run on an automated sequencer (ABI 377, Applied Biosystems).

### 2. Results

### Novel Polymorphisms

| ***Position*** | ***Reference*** | ***Region*** | ***Referenc e Allele*** | ***Second Allele*** | ***Effect*** | ***RFLP*** | ***Second Allele Frequency*** |
|---|---|---|---|---|---|---|---|
| 344 | SEQ ID NO.1 | | G | A | loss of SP1 site | | 3/54 |
| 621-627 | SEQ ID NO.1 | | 7xG | 6xG | | | 32/54 |
| 793-799 | SEQ ID NO.1 | | 7xG | 6xG | | | 5/52 |
| 908 | SEQ ID NO.1 | | C | T | loss of SP1 site | | 1/46 |
| 1136 | SEQ ID NO.1 | intron 1 | G | C | | | 11/34 |
| 1160 | SEQ ID NO.1 | intron 1 | T | C | | + eng Bfa I | 2/40 |
| 1189 | SEQ ID NO.1 | intron 1 | G | A | | | 12/40 |
| 1458 | SEQ ID NO.1 | intron 1 | A | G | | + eng Apa I | 19/38 |
| 1656 | SEQ ID NO.1 | intron 1 | T | G | | - eng Msc I | 2/40 |
| 2448 | SEQ ID NO.1 | exon 2 | T | C | Leul26Pro | + eng Sac II | 2/52 |
| 2531 | SEQ ID NO.1 | exon 2 | G | A | Alal54Thr | - Bss HI | 1/52 |
| 3348 | SEQ ID NO.1 | intron 2 | C | T | | | 17/50 |
| 3432 | SEQ ID NO.1 | intron 2 | C | G | | + Nla III | 7/40 |
| 3622 | SEQ ID NO.1 | exon 3 | G | A | silent polymorphis m | + eng Spe I | 12/38 |

Frequency is the allele frequency of the second allele in control subjects.

### Example 2

### Determination of the full length genomic DNA sequence of the human EP1-R gene

The full length genomic DNA sequence of the human EP1-R gene was obtained by PCR. Intron sequences were obtained by PCR from the flanking cDNA sequence. The genomic sequence 1-1073 was obtained by vectorette PCR (Riley *et al.,* Nuc. Acid Res. 18, 2887-2890, 1990).

### Comparison of corresponding regions of sequence in SEQ ID NO.1 and EMBL L22647

| *SEQ ID NO.1* *position number* | *EMBL L22647* *position number* |
|---|---|
| 1074-1130 | 1-57 |
| 2055-3013 | 58-1016 |
| 3566-3908 | 1017-1359 |

### Identification of errors in published EP1-R gene sequences

By carrying out a detailed comparison of the full length genomic DNA sequence of the EP1-R gene as defined in SEQ ID NO.1 with the cDNA sequence published in EMBL L22647 and the genomic DNA sequences published in EMBL AC008569, we have identified the following errors in the published sequences, some of which result in changes to the published amino acid sequence.

| ***Position as defined in L22647*** | ***Published sequence L22647*** | ***Sequence as determined in SEQ ID NO.1*** | ***Codon change*** | ***Amino acid change*** |
|---|---|---|---|---|
| 285 | A | G | ACC to GCC | Thr71A1a |
| 763 | A | T | CAT to CTA | His230Leu |
| 764 | T | A | | |

| ***Position as defined in SEQ ID NO.1*** | ***Published sequence AC008569*** | ***Sequence* as *determined in SEQ ID NO.1*** |
|---|---|---|
| 2912 | AA | A |
| 1088 | T | C |
| 2264 | A | C |
| 2275 | - | C |
| 2287 | A | C |
| 2699 | A | C |
| 2860 | A | C |
| 2985 | A | T |
| 3060 | T | C |
| 3062 | G | C |
| 3729 | A | G |

### Example 3

### Engineered RFLPs

| *Position* | *Diagnostic fragment* | *Forward Primer* | *Reverse Primer* |
|---|---|---|---|
| 1160 | 1082-1182 | 1082-1102 | 1161-1182 Bfa I |
| 1458 | 1082-1477 | 1082-1102 | 1459-1477 Apa I |
| 1656 | 1434-1682 | 1434-1453 | 1657-1682 Msc I |
| 2448 | 2351-2472 | 2351-2371 | 2449-2472 Sac II |
| 3622 | 3372-3646 | 3372-3395 | 3623-3646 Spe I |

All positions refer to the positions in SEQ ID NO.1. 
Primer 1161-1182 Bfa I
CTTCATGCCCTCCTCCTCCCTA SEQ ID NO.2
 C at position 1160 creates a Bfa I fragment in the diagnostic fragment. 
Primer 1459-1477 Apa I
CCTGCCCCATGGACGGGCC SEQ ID NO.3
 G at position 1458 creates an Apa I site in the diagnostic fragment. 
Primer 1657-1682 Msc I
TCTGATAGCTCTCACCCATTTTGGCC SEQ ID NO.4
 T at position 1656 creates a Msc I site in the diagnostic fragment. 
Primer 2449-2472 Sac II
TCCACGGCCATGCCACAGCCCCGC SEQ ID NO.5
 C at position 2448 creates a Sac II site in the diagnostic fragment. 
Primer 3623-3646 Spe I
GGAGGCAAGGCGCACGGCCAGGAC SEQ ID NO.6
 A at position 3622 creates a Spe I site in the diagnostic fragment.

## Claims

**1.** A method for the diagnosis of a polymorphism in an EP1-R gene in a human, which method comprises determining the sequence of the nucleic acid of the human at one or more of positions 344, 621-627, 793-799, 908, 1136, 1160, 1189, 1458, 1656, 2448, 2531, 3348, 3432 and 3622 in the EP1-R gene as defined by the positions in SEQ ID NO.1, or the sequence of the amino acid in the EP1-R protein at positions 126 or 154; and determining the status of the human by reference to polymorphism in the EP1-R gene or protein.

**2.** A method according to claim 1 wherein the polymorphism is selected from the group in which, according to the position in SEQ ID NO. 1, the polymorphism at position 344 is presence of G and/or A, the polymorphism at position 621-627 is deletion of one or more of the seven Gs, the polymorphism at position 793-799 is deletion of one or more of the seven Gs, the polymorphism at position 908 is presence of C and/or T, the polymorphism at position 1136 is presence of G and/or C, the polymorphism at position 1160 is presence of T and/or C, the polymorphism at position 1189 is presence of G and/or A, the polymorphism at position 1458 is presence of A and/or G, the polymorphism at position 1656 is presence of T and/or G, the polymorphism at position 2448 is presence of T and/or C, the polymorphism at position 2531 is presence of G and/or A, the polymorphism at position 3348 is presence of C and/or T, the polymorphism at position 3432 is presence of C and/or G and, the polymorphism at position 3622 is presence of G and/or A.

**3.** A method as claimed in claim 1 or 2, wherein the nucleic acid region containing the potential single nucleotide polymorphism is amplified by polymerase chain reaction prior to determining the sequence.

**4.** A method as claimed in any of claims 1 - 3, wherein the presence or absence of the single nucleotide polymorphism is detected by reference to the loss or gain of, optionally engineered, sites recognised by restriction enzymes.

**5.** A method according to claim 1 or claim 2, in which the sequence is determined by a method selected from ARMS-allele specific amplification, allele specific hybridisation, oligonucleotide ligation assay and restriction fragment length polymorphism (RFLP). A method according to claim 1 wherein the presence of a polymorphic amino acid residue in the EP1-R protein is determined by immunological methods such as enzyme linked immunosorbent assay (ELISA).

**6.** A method as claimed in any of the preceding claims for use in assessing the predisposition and/or susceptibility of an individual to EP1-R mediated diseases.

**7.** A method for the diagnosis of EP1-R-mediated disease, which method comprises:
i) obtaining sample nucleic acid from an individual,
ii) detecting the presence or absence of a variant nucleotide at one or more of positions 344, 621-627, 793-799, 908, 1136, 1160, 1189, 1458, 1656, 2448, 2531, 3348, 3432 and 3622 in the EP1-R gene as defined by the positions in SEQ ID NO.1; and
iii) determining the status of the individual by reference to polymorphism in the EP1-R gene.

**8.** A method for the diagnosis of EP1-R- mediated disease, which method comprises:
i) obtaining a protein containing sample from an individual;
ii) detecting the presence or absence of a variant EP1-R polypeptide on the basis of the presence of a polymorphic amino acid at either or both amino acid positions: 126 and 154; and,
iii) determining the status of the human by reference to the presence or absence of a polymorphism in EP1-R protein.

**9.** A nucleic acid comprising any one of the following polymorphisms:
the nucleic acid of SEQ ID NO.1 with A at position 344 as defined by the positions in SEQ ID NO.1; the nucleic acid of SEQ ID NO.1 with six Gs at positions 621-626 as defined by the positions in SEQ ID NO.1; the nucleic acid of SEQ ID NO.1 with six Gs at positions 793-798 as defined by the positions in SEQ ID NO.1; the nucleic acid of SEQ ID NO.1 with T at position 908 as defined by the positions in SEQ ID NO.1; the nucleic acid of SEQ ID NO.1 with C at position 1136 as defined by the positions in SEQ ID NO.1; the nucleic acid of SEQ ID NO.1 with C at position 1160 as defined by the positions in SEQ ID NO.1; the nucleic acid of SEQ ID NO.1 with A at position 1189 as defined by the positions in SEQ ID NO.1; the nucleic acid of SEQ ID NO.1 with G at position 1458 as defined by the positions in SEQ ID NO.1; the nucleic acid of SEQ ID NO.1 with G at position 1656 as defined by the positions SEQ ID NO.1; the nucleic acid of SEQ ID NO.1 with C at position 2448 as defined by the positions in SEQ ID NO.1; the nucleic acid of SEQ ID NO.1 with A at position 2531 as defined by the positions in SEQ ID NO.1; the nucleic acid of SEQ ID NO.1 with T at position 3348 as defined by the positions in SEQ ID NO.1; the nucleic acid of SEQ ID NO.1 with G at position 3432 as defined by the positions in SEQ ID NO.1; the nucleic acid of SEQ ID NO.1 with A at position 3622 as defined by the positions in SEQ ID NO.1; or a complementary strand thereof or an antisense sequence thereto or a fragment thereof of at least 17 bases comprising at least one polymorphism.

**10.** An allele specific primer or probe capable of detecting an EP1-R gene polymorphism at one or more of positions 344, 621-627, 793-799, 908, 1136, 1160, 1189, 1458, 1656, 2448, 2531, 3348, 3432, and 3622 in the EP1-R gene as defined by the positions in SEQ ID NO.1.

**11.** A diagnostic kit comprising one or more diagnostic primer(s) and/or probes(s) as defined in claim 10.

**13.** Use of an EP1-R drug in the preparation of a medicament for treating an EP1 R-mediated disease in a human diagnosed as having a polymorphism at one or more of positions 344, 621-627, 793-799, 908, 1136, 1160, 1189, 1458, 1656, 2448, 2531, 3348, 3432 and 3622 in the EP1-R gene as defined by the positions in SEQ ID NO.1.

**14.** A pharmaceutical pack comprising an EP1-R drug and instructions for administration of the drug to humans diagnostically tested for a polymorphism at one or more of positions 344, 621-627, 793-799, 908, 1136, 1160, 1189, 1458, 1656, 2448, 2531, 3348, 3432 and 3622 in the EP1-R gene as defined by the positions in SEQ ID NO.1.

**15.** A computer readable medium having stored thereon a nucleic acid sequence comprising at least 17, preferably at least 20 consecutive bases of the EP1-R gene sequence, which sequence includes at least one of the polymorphisms at positions: 344, 621-627, 793-799, 908, 1136, 1160, 1189, 1458, 1656, 2448, 2531, 3348, 3432 and 3622 in the EP1-R gene as defined by the positions in SEQ ID NO.1.

**16.** A method for performing sequence identification, said method comprising the steps of providing a nucleic acid sequence comprising at least 20 consecutive bases of the EP1-R gene sequence, which sequence includes at least one of the polymorphisms at positions: 344, 621-627, 793-799, 908, 1136, 1160, 1189, 1458, 1656, 2448, 2531, 3348, 3432 and 3622 in the EP1-R gene as defined by the positions in SEQ ID NO.1, in a computer readable medium; and comparing said nucleic acid sequence to at least one other nucleic acid sequence to identify identity.

**17.** A purified allelic variant of the human EP1-R polypeptide having a proline at position 126 and/or a threonine at position 154 or a fragment thereof comprising at least 10 amino acids provided that the fragment comprises the allelic variant at position 126 and/or position 154.

**18.** An antibody specific for an allelic variant of human EP1-R polypeptide having a proline at position 126 and/or a threonine at position 154 or a fragment thereof comprising at least 10 amino acids provided that the fragment comprises the allelic variants at position 126 and /or position 154.


SEQUENCE LISTING

<110> ASTRAZENECA AB
      ANAND, RAKESH
      SMITH, JOHN C
      MORTEN, JOHN E N

<120> DIAGNOSTIC METHOD

<130> LDSG/Z70667

<140>
<141>

<160> 6

<170> PatentIn Ver. 2.1

<210> 1
<211> 3918
<212> DNA
<213> Human

<400> 1
aaacagagtg tggtcagggg cagttttggc aataaggagt gtgtgtgcgt gtgtgtatct 60
ctcggggtgc caagtgagac cctatttccc agcactaagg agggtggttc gggtgatcat 120
ctcagagggc tgcattttgg ggtgttggga ggggctggaa ttgaggcagt aactctgggt 180
gcgtcccggc gctgggggta agaccctgag ttcagggatg tggcggcagc aacctggctg 240
tgctgagatg gggacaggat gaaactgagt ggtaagtgac aggattgtat gtcttagggt 300
gtgaggctgt gccagtgtga ccctacttct cagggcagga ggcgagtctt tgtgtcttag 360
gacgtgtgtc acaggtgtca ctcagcccat cttaaaggag ccagtcactg gggagagtcc 420
ccaggtcgcc aggcctcctg gttcccctgc cccctttcag cctcctccca ggatatgact 480
cgctgtggcc ctagccgggg cttaagcccc tgtataaata ggcggatccc ctggccaggc 540
tgggcctgac ccaagctggt ctacccgagg ccctgcccac cagcacccac cccgacatcc 600
acgaaggctt tggcagggca gggggggcat tgctggcccc acagaattgg agttagcctc 660
tctagctcca aagccccagg gaggggggca gtggcacccc ctgcagctgc cccagcccgc 720
cccagacgac gctcacattt tccagtgttt agattttatc cgctttatta atgaggcaag 780
aggcccgatc ctggggggga agggggctgc tcccgccctg ctgggaggag ggggtcacat 840
ggggccagac caactccagg gagcctcact cctcgtggag ggagccgctg gggcccagag 900
tggcccccgc ccattgccag gagcagagag gggggtctct gccactccag gcccccagcg 960
ggcgtgcgcg gggtggagcg gcccctggag gccagcaagg gcggcgggag gagctgagga 1020
gccgccccag gaagagggag ggaggaagcg gcttgccgga gagccagggc gcagtgggcg 1080

gcagggctga gcggccggtg atggggaccc cacatcccag gcagtgccgg gtaagcgggt 1140
tgcacatgag ggtccctgat tggggaggag gagggcatga aggggtgggg ccatggccac 1200
ctgctgtccg tcctgtgtcc ccgggctctt ctccctgatc tgtgcgtccc tgtcttaccg 1260
tctgtccatc ctcccttccc atctggcctg gtgggcagtt ccgccctttc agccctcacc 1320
accaggatat caaagagtgg atggtcgtgg ggccgcctcc aggcctctgg ggcagggata 1380
tggagtggaa ccgtgggcac ccagggtgag gcagaggtga tcggggtagg gcctggcgag 1440
agaccgcagt ggagcagggg caggtccatg gggcaggggc agagatgggt ccatggcaca 1500
gatgtggctg tgccatgtgg ggtcagagat gggtccatgg gggacaggga catggccgtg 1560
gggagagatg tgcccgtggt ggtagagatg gggctggggc atcgaggatg ggactggagt 1620
aaagctgaca tggggaaagt caggtagggc cacagtggca gaaatgggtg agagctatca 1680
gatggagcca caggccccag gaatttgctg ggtgtaaaaa tggaaggtgg gggtcggagg 1740
cactggcaga gatgcctgag ggcggggctg gggggaatct tgcaggaaac catgaagggc 1800
agagaaaggg ccagtggggt tagagggagg ccctggagca ggagatgggg tggtgagagg 1860
caaaagagag ggagaagggt ttccaaatgg agtggccagg tcatttggag ttgcccatgg 1920
caactgccat gggcagaggg gccgcctgag aacgccatgg agtcaaacag gccctgatgt 1980
tctgagatgg caccgtgggc tggtccccgc ccgggcccag ccagcctcac tctgccctcc 2040
tctcctctat ccagcacccc tggcgcctga catgagccct tgcgggcccc tcaacctgag 2100
cctggcgggc gaggcgacca catgcgcggc gccctgggtc cccaacacgt cggccgtgcc 2160
gccgtcgggc gcttcgcccg cgctgcccat cttctccatg acgctgggcg ccgtgtccaa 2220
cctgctggcg ctggcgctgc tggcgcaggc cgcgggccgc ctgcgacgcc gccgctcggc 2280
cgccaccttc ctgctgttcg tggccagcct gctggccacc gacctggcgg gccacgtgat 2340
cccgggcgcg ctggtgctgc gtctgtacac tgcggggcgc gctccggccg gcggggcctg 2400
ccacttcctg ggcggctgca tggtcttctt cggcctgtgc ccgctgctgc tgggctgtgg 2460
catggccgtg gagcgctgcg tgggcgtcac gcggccgctg ctccacgccg cgcgggtctc 2520
ggtcgcccgc gcgcgcctgg cgctggccgc ggtggccgcg gtggccttgg ccgtggcgct 2580
gctgccgctg gcgcgcgtgg gccgctatga gctgcagtac ccgggcacgt ggtgcttcat 2640
cggcctgggt cccccgggcg gctggcgcca ggcactgctt gctggcctct tcgccagcct 2700
cggcctggtc gcgctcctcg ccgcgctggt gtgcaacacg ctcagcggcc tggccctgct 2760
acgcgcccgc tggcgacgcc gctcccgacg gcctcccccg gcctcaggcc ccgacagccg 2820
gcgtcgctgg ggggcgcacg gaccccgctc ggcctccgcc tcgtccgcct cgtccatcgc 2880
ttcggcctcc accttctttg gcggctctcg gagcagcggc tcggcacgca gagctcgcgc 2940
ccacgacgtg gagatggtgg gccagcttgt cggtatcatg gtggtgtcgt gcatctgctg 3000
gagcccaatg ctggtgaggg gcgcaccggc ccctcgagcc acgctccttc ccgctccctc 3060
tcggcaccct cccgcccttt gtcgtcccag gacacctggg gcctccatcc tggactcaac 3120
caaggccccg gcccctagag gccccacctg ccccgaaagc cagcatcgcc ttctccatct 3180
gacctcccat ccttcctcct agcccccctc tcctcttcct ttttggggtc tttgtagcgc 3240
accccgaccc acacaagcct cctctcctgc cccaccgtta taagtcgccg cgcttcattc 3300
cctagtcctt tcacccaacc cccttgcttt tcctctttcc gggacacctg agactcctct 3360
acggccggac ccccacccac tgaaggtgtt tgtttctttg cccccctttt ttttccgcat 3420
ccgtttctca tctggatccc catttaccct ccctgcgacg gctcgcccct cctcccaggc 3480
ttttaccttc cagccacgcc cccaccatcc ctgcgccccc ctgcgcccgc gcctgctcta 3540
tagctcacac cggcttcccc cgcaggtgtt ggtggcgctg gccgtcggcg gctggagctc 3600

tacctccctg cagcggccac tgttcctggc cgtgcgcctt gcctcctgga accagatcct 3660
ggacccttgg gtgtacatcc tactgcgcca ggccgtgctg cgccaactgc ttcgcctctt 3720
gcccccgagg gccggagcca agggcggccc cgcggggctg ggcctaacac cgagcgcctg 3780
ggaggccagc tcgctgcgca gctcccggca cagcggcctc agccacttct aagcacaacc 3840
agaggcccaa cgactaagcc agcccaccct gggctgggcc caggtgcgcg gcgcagagcc 3900
tttgggaact tgggccag 3918

<210> 2
<211> 22
<212> DNA
<213> Artificial Sequence

<220>
<223> Description of Artificial Sequence: Primer

<400> 2
cttcatgccc tcctcctccc ta 22

<210> 3
<211> 19
<212> DNA
<213> Artificial Sequence

<220>
<223> Description of Artificial Sequence: Primer

<400> 3
cctgccccat ggacgggcc 19

<210> 4
<211> 26
<212> DNA
<213> Artificial Sequence

<220>
<223> Description of Artificial Sequence: Primer

<400> 4
tctgatagct ctcacccatt ttggcc 26

<210> 5

<211> 24

<212> DNA

<213> Artificial Sequence

<220>

<223> Description of Artificial Sequence: Primer

<400> 5

tccacggcca tgccacagcc ccgc 24

<210> 6

<211> 24

<212> DNA

<213> Artificial Sequence

<220>

<223> Description of Artificial Sequence: Primer

<400> 6

ggaggcaagg cgcacggcca ggac 24